# EUROPÄISCHE PATENTANMELDUNG

(11) **EP 4 706 631 A1**
(43) Veröffentlichungstag der Anmeldung: **11.03.2026**
(21) Anmeldenummer: 25196600.8
(22) Anmeldetag: 19.08.2025
(51) Int. Cl.: A61K 8/36, A61K 8/92, A61K 9/00, A61K 35/00, A61P 11/02, A61P 11/04, A61Q 11/00

(54) **HYGIENEMITTEL**

(30) Priorität: 29.08.2024 DE 102024002791
(71) Anmelder: Smeja, Ulrich, 45881 Gelsenkirchen (DE)
(72) Erfinder: Smeja, Ulrich, 45881 Gelsenkirchen (DE)
(74) Vertreter: Grosse Schumacher Knauer von Hirschhausen

(57) **Zusammenfassung**

Die Erfindung betrifft ein Hygienemittel für die Mund-, Rachen-, Nasen-, und/oder Atemwegs-Hygiene oder für die Haut-Hygiene umfassend einen Essig-Bestandteil. Das Hygienemittel umfasst als weiteren Bestandteil zumindest ein Öl und/oder ein ätherisches Öl, insbesondere ein Minzöl, Oreganoöl und/oder ein Eukalyptus-Öl. Das Hygienemittel umfasst als weiteren Bestandteil zumindest ein Heilpflanzenextrakt.

## Beschreibung

### GEBIET DER ERFINDUNG

Die Erfindung betrifft ein, insbesondere oral respektive nasal anwendbares, Hygienemittel gemäß dem Oberbegriff des Anspruchs 1. Demnach ist das Hygienemittel für die Mund-, Rachen-, Nasen-, und/oder Atemwegs-Hygiene oder für die Haut-Hygiene geeignet und es umfasst einen Essig-Bestandteil, beispielsweise eine (wässrige) Essiglösung. Das Hygienemittel hat eine antimikrobielle, antibakterielle, antimykotische und/oder antivirale Wirkung, sodass durch Anwendung des Hygienemittels im menschlichen Mund-, Rachen-, Nasen- und/oder Atemwegs-Bereich oder auf der Haut dort ansässige Bakterien, Pilze und/oder Viren reduzierbar respektive vollständig beseitigbar sind.

Die Erfindung betrifft außerdem ein Hygiene-(Mund-)Spray, insbesondere zur oralen Anwendung, gemäß Anspruch 13, sowie eine Hygiene-Tablette zur oralen Anwendung gemäß Anspruch 14, ein Hygiene-Bonbon zur oralen Anwendung, insbesondere zum Lutschen oder Kauen, gemäß Anspruch 15, eine Hygiene-Lösung zum, insbesondere nasalen, Inhalieren, gemäß Anspruch 16 und eine Hygiene-Salbe nach Anspruch 17.

Schließlich betrifft die Erfindung ein Verfahren zum Verbessern der Mund-, Rachen-, Nasen-, und/oder Atemwegs-Hygiene oder der Haut-Hygiene gemäß dem Oberbegriff des Anspruchs 18.

### TECHNOLOGISCHER HINTERGRUND

Das Anwenden von Essig oder essigbasierten Lösungen zum Verbessern beispielsweise der Mundhygiene ist in der Naturheilkunde hinlänglich bekannt. Problematisch dabei ist, dass Essig-Anwendungen geschmacklich oder olfaktorisch oft als unbeliebt gelten, wodurch essigbasierte Hygieneprodukte insbesondere für Mundanwendungen regelmäßig gemieden oder gänzlich vermieden werden.

### DARSTELLUNG DER ERFINDUNG

Die der Erfindung zugrunde liegende Aufgabe besteht darin, die Essig- respektive Essiglösung-basierten Hygienemaßnahmen für den menschlichen Mund-, Rachen-, Nasen- und Atemwegsbereich oder für die Haut so zu optimieren, dass die Hygieneanwendung von den anwendenden Personen (Anwendern) als angenehm, gesund, nachhaltig und nebenwirkungsfrei empfunden werden. Diese Aufgabe wird durch ein Hygienemittel nach Anspruch 1 gelöst. Weitere Ausgestaltungen sind Gegenstand der Unteransprüche. Bei dem Hygienemittel ist demnach vorgesehen, dass das Hygienemittel als weiteren Bestandteil zumindest ein Öl und/oder ein ätherisches Öl umfasst, insbesondere ein Minzöl, Oreganoöl und/oder ein Eukalyptus-Öl. Durch die Öl-Zutat nimmt der Anwender den (strengen respektive bitteren) Essig-Geschmack und -Geruch weniger wahr und es entsteht der Eindruck, dass das Hygienemittel frisch oder würzig, insbesondere wohlschmeckend, wohlriechend und gesund ist. Zudem entsteht bei oraler Anwendung ein frischer respektive wohlriechender Atem, was vom Anwender selbst regelmäßig als angenehm verbunden wird, und was bei anderen Personen, die beispielsweise mit dem Anwender sprechen, einen gepflegten und hygienischen Eindruck vermittelt. Der (ätherische) Öl-Bestandteil wirkt im menschlichen Mund-/Nasen-/Rachen-Atemwegs-Bereich oder auf der (Schleim-)Haut zusätzlich entzündungshemmend und entkrampfend und entfacht dort eine zusätzliche antimikrobielle Aktivität, bei Eukalyptusöl beispielsweise gegen Bakterien wie E. coli, Staphylococcus aureus, Streptococcus faecalis, und Mycobacterium avium. Der (ätherische) Öl-Bestandteil wirkt außerdem fungizid, beispielsweise bei Eukalyptusöl gegen Candida tropicalis, Candida albicans und Aspergillus niger. Ein Minzöl im (äthereschen) Öl-Bestandteil des Hygienemittels kann zusätzlich eine analgetische Wirkung entfachen, sodass die anderen Bestandteile vom Anwender besser vertragen werden. Das Minzöl kann ferner den Eindruck einer kühlenden Wirkung entstehen lassen, insbesondere aufgrund des Methanol-Anteils im Minzöl. Das Hygienemittel ist auch dazu geeignet, eine Wahrscheinlichkeit für Virusinfektionen, insbesondere solche, die, beispielsweise im Mund-/Lippenbereich Hautläsionen verursachen, zu reduzieren und etwaige Viren zu neutralisieren.

Das Hygienemittel umfasst als weiteren Bestandteil zumindest ein Pflanzenextrakt und/oder ein Heilpflanzenextrakt, vorzugsweise ein (flüssiges) Salbeiextrakt, ein (flüssiges) Spitzwegerichextrakt, ein (flüssiges) Königskerzenextrakt und/oder ein (flüssiges) Alantextrakt. Das (Heil-)Pflanzenextrakt respektive die (Heil-)Pflanzenex-trakte kann/können einen Stabilisator umfassen und/oder Wasser. Salbei hilft unter anderem dabei, Entzündungen, insbesondere im Mund- und Rachen-Bereich, zu lindern, was die Wirkung des hierin beschriebenen und beanspruchten Hygienemittels verbessert. Es wird insbesondere die Mundhygiene verbessert. Spitzwegerich, der auch Heilwegerich oder Wundwegerich genannt wird, wirkt unter anderem reizmildernd, antibakteriell und entzündungshemmend. Er wirkt beispielsweise gegen entzündliche Veränderungen der Mund- und Rachenschleimhaut. Er kann auch gegen entzündliche Veränderungen der Haut eingesetzt werden, beispielsweise bei Insektenstichen. Diese Eigenschaften verbessern die Wirkung des hierin beschriebenen und beanspruchten Hygienemittels erheblich. Königskerzen wirken reizmildernd und expektorierend (lösend) etwa bei Katarrhen (z. B Husten). (Echter) Alant wirkt heilend, z. B. bei wunden (Schleim-)Hautstellen und verbessert ebenfalls die Wirkung des hierin beschriebenen und beanspruchten Hygienemittels. Der Geschmack respektive Geruch von Salbei/Alant/Königskerzen/Spitzwegerich oder Mischungen daraus wird von vielen Anwendern als wohlschmeckend, wohlriechend, natürlich und gesund wahrgenommen, insbesondere wenn das (Heil-)Pflanzenextrakt ausschließlich aus Naturprodukten gewonnen wird. Insbesondere die Kombination aus (Bio-) Apfelessig, Salbeiextrakt, Spitzwegerichextrakt, Königskerzenextrakt, Alantextrakt, Eukalyptusöl und Menthol und ggf. Oreganoöl erwies sich in Tests und Versuchen als effizientes, die (Mund-/Haut-)Hygiene verbesserndes Mittel bei gleichzeitig angenehmem Geschmack und Geruch. Ein die Hygiene verbesserndes Mittel ohne angenehmen Geschmack wird von den Testpersonen überwiegend abgelehnt. Ein die Hygiene verbesserndes Mittel, das nur gut schmeckt, jedoch die Hygiene nur unwesentlich beeinflusst, wird von den Testpersonen ebenfalls abgelehnt. Insofern leistet die hierin beschriebene und beanspruchte Kombination aus natürlichen, die Hygiene verbessernden Bestandteilen (z. B. Apfelessig + Salbei + Spitzwegerich + Alant + Eukalyptusöl + Menthol, ggf. + Oreganoöl, alles in jeweils zweckmäßigen Mengen respektive Anteilen) bei gleichzeitig angenehmem Geschmack/Geruch einen wichtigen Beitrag zur Hygieneverbesserung beim Anwender. Dass die genannten Bestandteile dabei ausschließlich natürlicher Herkunft sind und kein Pharmazeutikum bilden, stieß bei allen Testpersonen auf großen Zuspruch und viel Anerkennung. So weiß der Anwender, dass das wohlschmeckende und wohlriechende Mittel zur effizienten Hygieneverbesserung gesund und dessen Anwendung für den Anwender bei vernünftiger Dosierung unschädlich ist.

Das hierin beschriebene Hygienemittel besteht ausschließlich aus Bestandteilen natürlichen Ursprungs und es umfasst keine chemischen oder pharmazeutischen Erzeugnisse. Die (orale oder nasale oder Haut-) Anwendung des hierin beschriebenen Hygienemittels ist (nahezu) nebenwirkungsfrei (sofern der Anwender nicht allergisch auf die Inhaltsstoffe reagiert).

Der Anteil des Pflanzenextrakt-Bestandteils oder des Heilpflanzenextrakt-Bestandteils beträgt bevorzugt mindestens etwa 10 Vol.%. Der Anteil des Pflanzenextrakt-Bestandteils oder des Heilpflanzenextrakt-Bestandteils kann vorzugsweise in einem Bereich zwischen etwa 15 Vol.% und etwa 40 Vol.% liegen.

Gemäß einer Ausgestaltung der Zusammensetzung des Hygienemittels kann vorgesehen sein, dass das Hygienemittel als weiteren Bestandteil Menthol umfasst. Es kann beispielsweise ein Menthol-Öl als weiterer Bestandteil vorgesehen sein. Der ein Anteil an ätherischem Öl oder ätherischen Ölen, sofern vorgesehen, kann kleiner ist als 0,50 Vol.% sein, insbesondere zwischen 0,00 Vol.% und 0,49 Vol.%. Der Menthol-Anteil, sofern vorgesehen kann kleiner sein als 0,50 Vol.%, insbesondere zwischen 0,00 Vol.% und 0,49 Vol.%. Die Summe der Anteile an Methanol und ätherischem Öl oder ätherischen Ölen, sofern jeweils vorgesehen, kann kleiner sein als 0,50 Vol.%, insbesondere zwischen 0,00 Vol.% und 0,49 Vol.%. Das hierin beschriebene und beanspruchte Hygienemittel ist somit als "alkoholfrei" kennzeichenbar, insbesondere im lebensmittelrechtlichen respektive im kosmetikrechtlichen Kontext.

Gemäß einer weiteren Ausgestaltung kann vorgesehen sein, dass das Hygienemittel als weiteren Bestandteil zumindest ein Aroma umfasst, insbesondere ein Zitronenmelisse-Aroma, ein Erdbeer-Aroma, ein Beeren- oder Beerenmix-Aroma, ein Limetten-Aroma, ein Honig-Aroma und/oder ein Minze-Aroma. Durch den Aroma-Bestandteil wird der Geschmack und der Geruch des Mittels verbessert, was dazu führt, dass der Anwender das Hygienemittel häufiger anwendet, wodurch die (Mund-/Nasen-/Rachen-/Atemwegs-)Hygiene kontinuierlich verbessert wird. Es kann für den Geschmack/Geruch zweckmäßig sein, wenn das Hygienemittel als weiteren Bestandteil ein Süßungsmittel umfasst, insbesondere einen Zucker, einen Süßstoff, einen pflanzlichen Süßstoff und/oder Stevia.

Der Essig-Bestandteil des Hygienemittels kann Zitronenmelisse-Essig, Brombeer-Essig, Apfel-Essig, Branntwein-Essig, Balsamico-Essig, Reis-Essig und/oder Honig-Essig umfassen oder daraus gebildet sein. Bevorzugt wird Bio-Essig verwendet. Der Essig-Bestandteil kann einen Säureanteil von weniger als etwa 15 Vol.% haben, vorzugsweise einen Säureanteil zwischen etwa 3 Vol.% und etwa 8 Vol.%. Gemäß einer besonders bevorzugten Variante beträgt der Säureanteil etwa 5 Vol.%. Der Anteil des Essig-Bestandteils des Hygienemittels beträgt beispielsweise mindestens etwa 30 Vol.%, wobei der Anteil des Essig-Bestandteils vorzugsweise im Bereich zwischen etwa 40 Vol.% und etwa 95 Vol.% liegt.

Der Anteil des Öl-Bestandteils oder des ätherischen Öl-Bestandteils des Hygienemittels beträgt beispielsweise mindestens etwa 2 Vol.%, wobei der Anteil des Öl-Bestandteils oder des ätherischen Öl-Bestandteils besonders bevorzugt in einem Bereich zwischen etwa 3 Vol.% und etwa 8 Vol.% liegt. Gemäß einer besonders bevorzugten Variante beträgt der Anteil des (ätherischen) Öl-Bestandteils etwa 5 Vol.%.

Als weiterer Bestandteil des Hygienemittels können vorgesehen sein: Honig mit konservierender und entzündungshemmender Wirkung, Dattelsirup mit süßender Wirkung und fruchtigem Eigengeschmack/-geruch, Agavendicksaft mit antibakterieller Wirkung, Erythrit mit süßender Wirkung, Karamell mit süßender Wirkung und zur Geschmacks- respektive Geruchsveränderung, Vanille zur Geschmacks- respektive Geruchsveränderung und/oder Ingwer mit entzündungshemmender und antiviraler Wirkung.

Ein Optimieren von Essig- respektive Essiglösung-basierten Hygienemaßnahmen für den menschlichen Mund-, Rachen-, Nasen- und Atemwegsbereich oder für die Haut gelingt ferner mit einem Hygiene-(Mund-)Spray gemäß Anspruch 13. Das Hygiene-(Mund-)Spray umfasst ein hierin beschriebenes und beanspruchtes Hygienemittel oder ist daraus gebildet. Gemäß einer bevorzugten Variante des Hygiene-(Mund-) Sprays kann vorgesehen sein, dass das Spray etwa 60% (Bio-Apfel-)Essig umfasst und etwa 5 Vol.% (Eukalyptus-)Öl. Als weitere Bestandteile kommen in Betracht: Ein Salbei-Extrakt (beispielsweise mit einem Anteil von etwa 30 Vol.%) und ein Süßstoff (etwa Stevia, beispielsweise mit einem Anteil von etwa 5 Vol.%).

Ein Optimieren von Essig- respektive Essiglösung-basierten Hygienemaßnahmen für den menschlichen Mund-, Rachen-, Nasen- und Atemwegsbereich oder für die Haut gelingt außerdem mit einer Hygiene-Tablette gemäß Anspruch 14. Die Hygiene-Tablette zur oralen Anwendung umfasst ein hierin beschriebenes und beanspruchtes Hygienemittel. Die Tablette wird vom Anwender zerkaut wobei sich die Bestandteile im Mund auflösen und dort die Hygiene-Wirkung entfalten.

Ein Optimieren von Essig- respektive Essiglösung-basierten Hygienemaßnahmen für den menschlichen Mund-, Rachen-, Nasen- und Atemwegsbereich oder für die Haut gelingt auch mit einem Hygiene-Bonbon gemäß Anspruch 15. Das Bonbon wird vom Anwender gelutscht oder gekaut und umfasst ein hierin beschriebenes und beanspruchtes Hygienemittel. Gemäß einer bevorzugten Ausgestaltung umfasst das Hygiene-Bonbon einen Anteil von etwa 40 Gew.% (Bio-Apfel-)Essig und einen Anteil von etwa 3 Gew.% (Eukalyptus-)Öl. Es kann ein Zuckeranteil von etwa 40 Gew.% vorgesehen sein und ein Salbei-Extrakt-Anteil von etwa 17 Gew.%.

Ein Optimieren von Essig- respektive Essiglösung-basierten Hygienemaßnahmen für den menschlichen Mund-, Rachen-, Nasen- und Atemwegsbereich oder für die Haut gelingt gemäß Anspruch 16 auch mit einer Hygiene-Lösung zum, insbesondere nasalen, Inhalieren. Die Hygiene-Lösung umfasst ein hierin beschriebenes und beanspruchtes Hygienemittel oder ist daraus gebildet. Gemäß einer bevorzugten Ausgestaltung umfasst die Hygiene Lösung einen Anteil an (Bio-Apfel-)Essig von etwa 90 Vol.% oder von weniger als etwa 90 Vol.% und einen Öl-Anteil von etwa 10 Vol.% oder weniger als etwa 10 Vol.%. Es kann vorgesehen sein, dass die Lösung einen Eukalyptus-Öl-Anteil von etwa 5 Vol.% oder weniger als etwa 5 Vol.% umfasst und einen Menthol-Öl-Anteil von etwa 5 Vol.% oder weniger als etwa 5 Vol.%.

Ein Optimieren von Essig- respektive Essiglösung-basierten Hygienemaßnahmen für den menschlichen Mund-, Rachen-, Nasen- und Atemwegsbereich oder für die Haut gelingt gemäß Anspruch 16 ferner mit einer Hygiene-Salbe zum Auftragen auf die Haut oder auf die Lippen. Die Hygiene-Salbe umfasst ein hierin beschriebenes und beanspruchtes Hygienemittel.

Ein Optimieren von Essig- respektive Essiglösung-basierten Hygienemaßnahmen für den menschlichen Mund-, Rachen-, Nasen- und Atemwegsbereich oder für die Haut gelingt schließlich gemäß Anspruch 18 mit einem Verfahren zur Verbesserung der Mund-, Rachen-, Nasen-, und/oder Atemwegs-Hygiene. Dabei wird ein hierin beschriebenes und beanspruchtes Hygienemittel in den Mund-, Nasen- Rachen- oder Atemwegsbereich oder für die Haut verbracht. Dies geling beispielsweise dadurch, dass, ein hierin beschriebenes und beanspruchtes Hygiene-Mundspray angewendet wird, oder dass eine hierin beschriebene und beanspruchte Hygiene-Tablette verabreicht und zerkaut wird, oder dass ein hierin beschriebenes und beanspruchtes Hygiene-Bonbon gelutscht oder gekaut wird, oder dass eine hierin beschriebene und beanspruchte Hygiene-Lösung, insbesondere nasal, inhaliert wird, oder dass eine hierin beschriebene und beanspruchte Hygiene-Salbe, beispielsweise auf die Lippen, aufgetragen wird.

Die vorgenannten sowie die beanspruchten und in den Ausführungsbeispielen beschriebenen erfindungsgemäß zu verwendenden Bestandteile unterliegen in ihrer Konzeption keinen besonderen Ausnahmebedingungen, so dass die in dem Anwendungsgebiet bekannten Auswahlkriterien uneingeschränkt Anwendung finden können.

## Patentansprüche

1. Hygienemittel für die Mund-, Rachen-, Nasen-, und/oder Atemwegs-Hygiene oder für die Haut-Hygiene umfassend einen Essig-Bestandteil, insbesondere Apfelessig-Bestandteil **wobei** das Hygienemittel als weiteren Bestandteil zumindest ein Öl und/oder ein ätherisches Öl umfasst, insbesondere ein Minzöl, ein Oreganoöl und/oder ein Eukalyptus-Öl, **dadurch gekennzeichnet, dass** das Hygienemittel als weiteren Bestandteil zumindest ein Heilpflanzenextrakt umfasst.

2. Hygienemittel nach Anspruch 1, **dadurch gekennzeichnet, dass** das Heilpflanzenextrakt Salbeiextrakt, Spitzwegerichextrakt, Königskerzenextrakt, und/oder Alantextrakt umfasst oder daraus gebildet ist.

3. Hygienemittel nach Anspruch 1 oder 2, **dadurch gekennzeichnet, dass** der Anteil des Pflanzenextrakt-Bestandteils oder des Heilpflanzenextrakt-Bestandteils mindestens etwa 10 Vol.% beträgt, wobei der Anteil des Pflanzenextrakt-Bestandteils oder des Heilpflanzenextrakt-Bestandteils vorzugsweise in einem Bereich zwischen etwa 15 Vol.% und etwa 40 Vol.% liegt.

4. Hygienemittel nach einem der Ansprüche 1 bis 3, **dadurch gekennzeichnet, dass** das Hygienemittel als weiteren Bestandteil Menthol.

5. Hygienemittel nach einem der Ansprüche 1 bis 4, **dadurch gekennzeichnet,**
**dass** ein Anteil an ätherischem Öl oder ätherischen Ölen kleiner ist als 0,50 Vol.%, insbesondere zwischen 0,00 Vol.% und 0,49 Vol.%, oder
**dass** der Menthol-Anteil kleiner ist als 0,50 Vol.%, insbesondere zwischen 0,00 Vol.-% und 0,49 Vol.%, oder
**dass** die Summe der Anteile an Methanol und ätherischem Öl oder ätherischen Ölen kleiner ist als 0,50 Vol.%, insbesondere zwischen 0,00 Vol.% und 0,49 Vol.%,
sodass das Hygienemittel als "alkoholfrei" kennzeichenbar ist.

6. Hygienemittel nach einem der Ansprüche 1 bis 5, **dadurch gekennzeichnet, dass** das Hygienemittel als weiteren Bestandteil zumindest ein Aroma umfasst, insbesondere ein Zitronenmelisse-Aroma, ein Erdbeer-Aroma, ein Beeren- oder Beerenmix-Aroma, ein Limetten-Aroma, ein Honig-Aroma und/oder ein Minze-Aroma.

7. Hygienemittel nach einem der Ansprüche 1 bis 6, **dadurch gekennzeichnet, dass** das Hygienemittel als weiteren Bestandteil ein Süßungsmittel umfasst, insbesondere einen Zucker, einen Süßstoff, einen pflanzlichen Süßstoff und/oder Stevia.

8. Hygienemittel nach einem der Ansprüche 1 bis 7, **dadurch gekennzeichnet, dass** der Essig-Bestandteil Zitronenmelisse-Essig, Brombeer-Essig, Branntwein-Essig, Balsamico-Essig, Reis-Essig und/oder Honig-Essig umfasst oder daraus gebildet ist.

9. Hygienemittel nach einem der Ansprüche 1 bis 8, **dadurch gekennzeichnet, dass** der Essig-Bestandteil einen Säureanteil von weniger als etwa 15 Vol.% hat, vorzugsweise einen Säureanteil zwischen etwa 3 Vol.% und etwa 8 Vol.%.

10. Hygienemittel nach einem der Ansprüche 1 bis 9, **dadurch gekennzeichnet, dass** der Anteil des Essig-Bestandteils des Hygienemittels mindestens etwa 30 Vol.% beträgt, wobei der Anteil des Essig-Bestandteils vorzugsweise im Bereich zwischen etwa 40 Vol.% und etwa 95 Vol.% liegt.

11. Hygienemittel nach einem der Ansprüche 1 bis 10, **dadurch gekennzeichnet, dass** der Anteil des Öl-Bestandteils oder des ätherischen Öl-Bestandteils mindestens etwa 2 Vol.% beträgt, wobei der Anteil des Öl-Bestandteils oder des ätherischen Öl-Bestandteils vorzugsweise in einem Bereich zwischen etwa 3 Vol.% und etwa 8 Vol.% liegt.

12. Hygienemittel nach einem der Ansprüche 1 bis 11, **dadurch gekennzeichnet, dass** das als weiterer Bestandteil des Hygienemittels vorgesehen sind Honig, Dattelsirup, Agavendicksaft, Erythrit, Karamell, Vanille und/oder Ingwer.

13. Hygiene-Spray, insbesondere -Mundspray, insbesondere zur oralen Anwendung, **dadurch gekennzeichnet, dass** das Hygiene-Spray, insbesondere -Mundspray, ein Hygienemittel nach einem der Ansprüche 1 bis 12 umfasst oder daraus gebildet ist.

14. Hygiene-Tablette zur oralen Anwendung, **dadurch gekennzeichnet, dass** die Hygiene-Tablette ein Hygienemittel nach einem der Ansprüche 1 bis 12 umfasst.

15. Hygiene-Bonbon zur oralen Anwendung, insbesondere zum Lutschen oder Kauen, **dadurch gekennzeichnet, dass** das Hygiene-Bonbon ein Hygienemittel nach einem der Ansprüche 1 bis 12 umfasst.

16. Hygiene-Lösung zum, insbesondere nasalen, Inhalieren, **dadurch gekennzeichnet, dass** die Hygiene-Lösung ein Hygienemittel nach einem der Ansprüche 1 bis 12 umfasst oder daraus gebildet ist.

17. Hygiene-Salbe zum Auftragen auf die Haut oder auf die Lippen, **dadurch gekennzeichnet, dass** die Hygiene-Salbe ein Hygienemittel nach einem der Ansprüche 1 bis 12 umfasst.

18. Verfahren zur Verbesserung der Mund-, Rachen-, Nasen-, und/oder Atemwegs-Hygiene oder der Haut-Hygiene, **dadurch gekennzeichnet, dass** ein Hygienemittel nach einem der Ansprüche 1 bis 12 in den Mund-, Nasen- Rachen- oder Atemwegsbereich oder auf die Haut verbracht wird, indem ein Hygiene-Spray oder -Mundspray nach Anspruch 13 angewendet wird, oder indem eine Hygiene-Tablette nach Anspruch 14 verabreicht wird, oder indem ein Hygiene-Bonbon nach Anspruch 15 gelutscht oder gekaut wird, oder indem eine Hygiene-Lösung nach Anspruch 16, insbesondere nasal, inhaliert wird, oder indem eine Hygiene-Salbe nach Anspruch 17 aufgetragen wird.
